(19) Europäisches Patentamt
European Patent Office
Office européen des brevets

(11) **EP 3 560 527 B1**

(12) **EUROPEAN PATENT SPECIFICATION**

(45) Date of publication and mention
of the grant of the patent:
**31.01.2024 Bulletin 2024/05**

(21) Application number: **17881229.3**

(22) Date of filing: **15.12.2017**

(51) International Patent Classification (IPC):
**A61L 27/38** (2006.01)    **A61K 35/28** (2015.01)
**A61P 1/16** (2006.01)    **A61P 43/00** (2006.01)
**A61L 27/54** (2006.01)

(52) Cooperative Patent Classification (CPC):
**A61L 27/3834; A61K 35/28; A61L 27/3895;**
**A61L 27/54; A61P 1/16; A61P 43/00;**
A61L 2300/412; A61L 2300/414; A61L 2300/426;
A61L 2300/43

(86) International application number:
**PCT/JP2017/045037**

(87) International publication number:
**WO 2018/110686 (21.06.2018 Gazette 2018/25)**

(54) **TISSUE HEALING AGENT**

WIRKSTOFF FÜR GEWEBEHEILUNG

AGENT DE CICATRISATION DES TISSUS

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **16.12.2016 JP 2016244724**

(43) Date of publication of application:
**30.10.2019 Bulletin 2019/44**

(73) Proprietor: **Regene Pharm Co., Ltd.**
**Higashiosaka-shi, Osaka 577-0058 (JP)**

(72) Inventors:
• **MATSUYAMA, Akifumi**
**Higashiosaka-shi**
**Osaka 577-0058 (JP)**
• **OKURA, Hanayuki**
**Higashiosaka-shi**
**Osaka 577-0058 (JP)**

(74) Representative: **Moré, Solveig Helga et al**
**Kroher Strobel**
**Rechts- und Patentanwälte PartmbB**
**Bavariaring 20**
**80336 München (DE)**

(56) References cited:
WO-A1-2005/038014    WO-A1-2016/053758
WO-A2-2008/060374    JP-A- 2005 508 393
JP-A- 2008 307 205    JP-A- 2008 542 339
JP-A- 2011 525 355    JP-A- 2013 027 394
JP-A- 2015 507 921    JP-A- 2016 504 324
US-A1- 2008 019 944    US-A1- 2012 276 044
US-A1- 2014 286 911

• CRISOSTOMO, P. R. et al.: "Human mesenchymal
stem cells stimulated by TNF-alpha, LPS, or
hypoxia produce growth factors by an NFK B- but
not JNK-dependent mechanism", American
Journal of Physiology, vol. 294, March 2008
(2008-03), pages C675-C682, XP055493060,
• TI, D. D. et al.: "Lipopolysaccharides priming
mesenchymal stem cells accelerate diabetic WO
und healing via exosomes", Medical Journal of
Chinese People's Liberation Army, vol. 41, no. 7,
1 July 2016 (2016-07-01), pages 528-533,
XP055516583, ISSN: 0577-7402, DOI:
10.11855/j.issn.0577-7402.2016.07.02

• XIE, C. et al.: "Abstrac : PP15-18 Bone Marrow Mesenchymal Stem Cells Regulate Hepatic Stellate Cells Activation Through LPS-TLR4 Pathway", Hepatology International. The 21st Conference of the Asian Pacific Associationfor the Study of the Liver, vol. 5, no. 1, 3 February 2011 (2011-02-03), pages 290-291, XP055516590, ISSN: 1936-0533, DOI: 10.1007/s12072-010-9241-z

Remarks:

The file contains technical information submitted after the application was filed and not included in this specification

**Description**

Technical Field

The present invention relates to a pharmaceutical composition for tissue healing, and a method for producing the same. In particular, the present invention relates to a tissue healing agent containing drug-treated adherent cells derived from mesenchymal tissue, and a method for producing the same.

Background Art

Mesenchymal tissue-derived cells have been shown to be useful for tissue healing. Among them, mesenchymal stem cells (MSCs) are being actively studied for their clinical application in regenerative medicine. For example, tissue is considered as a source of stem cells (ASCs) (Non-Patent Document 1), and ASCs are known to have therapeutic effects in various areas (Non-Patent Document 2). In addition, adipose tissue-derived multilineage progenitor cells (ADMPCs) have also been shown to be effective for treatment of liver diseases (Patent Document 1).

Thus, mesenchymal tissue-derived cells have been shown to be useful in regenerative medicine involving tissue healing. Accordingly, further improvement of their healing ability is desired.

Patent Document 2 describes a purified population of mesenchymal stem cells that has been contacted with one or more of TNF-alpha, IL-1, and LPS under conditions to express increased levels of TSG-6. The cells are administered in combination with a carrier to a mammalian subject to reduce cardiac muscle cell necrosis.

Prior Art Documents

Patent Document

Patent Document 1: WO 2008/153179
Patent Document 2: WO 2009/154770

Non-Patent Documents

Non-Patent Document 1: Zuk PA, Zhu M, Ashjian P, et al. Human adipose tissue is a source of multipotent stem cells. Mol Biol Cell 2002; 13: 4279-4295.
Non-Patent Document 2: Japanese Journal of Transfusion and Cell Therapy, Vol. 59, No. 3: 450-456, 2013

Summary of the Invention

Problems to be Solved by the Invention

It is an object to further improve the tissue healing ability of mesenchymal tissue-derived cells.

Solutions to the Problems

As a result of intensive studies to solve the above problems, the present inventors have found that adherent cells derived from mesenchymal tissue treated with a physiologically active polypeptide or LPS (lipopolysaccharide) have extremely high tissue healing ability, and the present invention has been completed.

The present invention is defined by the independent claims, while the dependent claims concern the preferred embodiments. Any "embodiment" or "example" which is disclosed in the description but is not covered by the claims should be considered as presented for illustrative purpose only. The present invention provides the following:

A pharmaceutical composition for tissue healing, including adherent cells derived from mesenchymal tissue treated with a physiologically active polypeptide or LPS, and a pharmaceutically acceptable carrier.

The physiologically active polypeptide is interleukin-1 alpha (IL-1$\alpha$) or interleukin-1 beta (IL-1$\beta$).

The adherent cells derived from mesenchymal tissue are adipose tissue-derived multilineage progenitor cells (ADMPCs).

Preferably, the tissue healing is tissue protection, repair of tissue/cell injury, promotion of proliferation of cells constituting a tissue, suppression of tissue inflammation, wound healing or reconstruction of tissue form.

Preferably, the tissue healing is tissue healing in chronic phase disease.

The tissue to be treated preferably is liver tissue or cardiac tissue.

Also disclosed herein is a method for producing a pharmaceutical composition for tissue healing in chronic phase disease, including the steps of:

(a) treating adherent cells derived from mesenchymal tissue with a physiologically active polypeptide or LPS, and

(b) mixing the cells treated in step (a) with a pharmaceutically acceptable carrier.

The physiologically active polypeptide is interleukin-1 alpha (IL-1$\alpha$) or interleukin-1 beta (IL-1$\beta$)

The adherent cells derived from mesenchymal tissue are adipose tissue-derived multilineage progenitor cells (ADMPCs). Preferably, the tissue healing is tissue protection, repair of tissue/cell injury, promotion of proliferation of cells constituting a tissue, suppression of tissue inflammation, wound healing or reconstruction of tissue form.

It is further preferred that the tissue is liver tissue or cardiac tissue.

Effects of the Invention

According to the present invention, a pharmaceutical composition having an extremely high tissue healing ability can be obtained. The pharmaceutical composition of the present invention is useful for tissue healing in chronic phase-tissue injury and the like.

Brief Description of Drawings

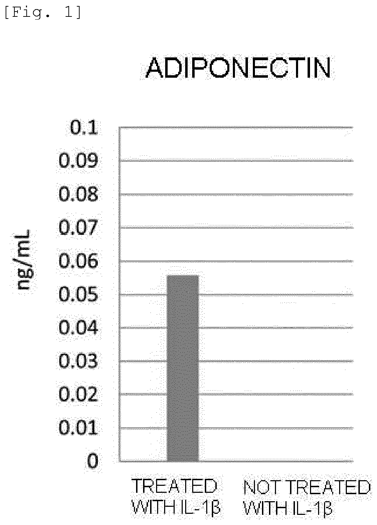

Fig. 1 is a graph comparing the produced amount of adiponectin in adipose tissue-derived multilineage progenitor cells (ADMPCs) treated with IL-1$\beta$ (left) and the produced amount of adiponectin in ADMPCs not treated with IL-1$\beta$ (right). The vertical axis represents the amount of adiponectin produced.

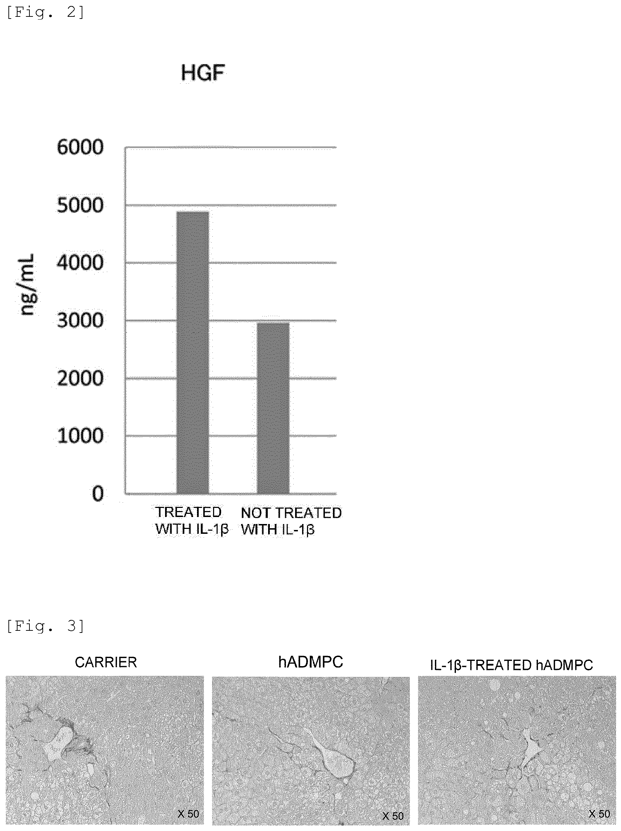

Fig. 2 is a graph comparing the produced amount of hepatocyte growth factor (HGF) in ADMPCs treated with IL-1$\beta$ (left) and the produced amount of HGF in ADMPCs not treated with IL-1$\beta$ (right). The vertical axis represents the amount of HGF produced.

Fig. 3 is an image of a Sirius red-stained tissue section showing decrease in intrahepatic fibers by ADMPCs treated with IL-1$\beta$ in non-alcoholic steatohepatitis (NASH) model mice. The result of a carrier administered group is in the left, the result of an administrated group with ADMPCs not treated with IL-1$\beta$ is in the middle, and the result of an administered group with ADMPCs treated with IL-1$\beta$ is in the right. The magnification is 50 times.

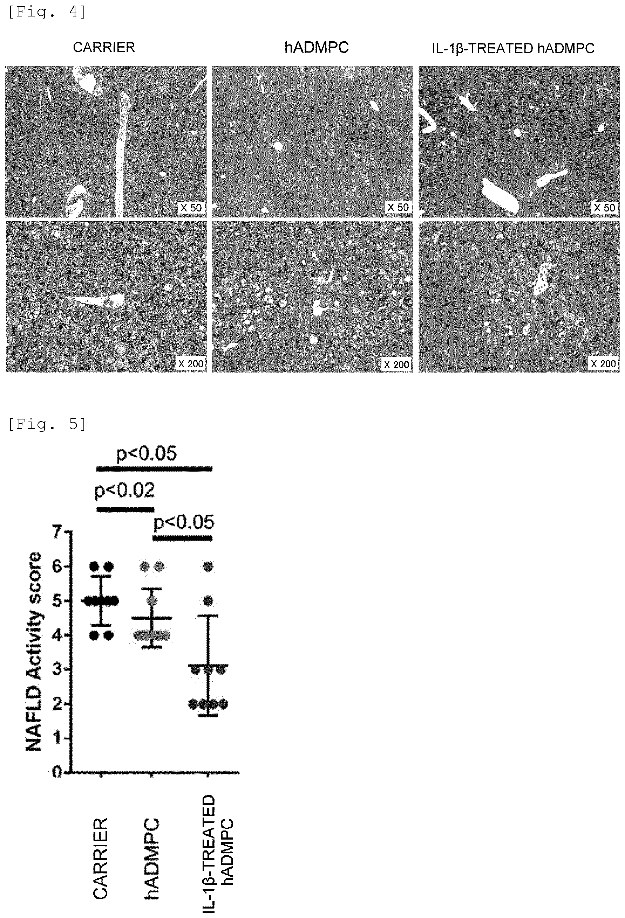

Fig. 4 is an image of a HE-stained tissue section showing reduction in liver tissue injury by ADMPCs treated with IL-1$\beta$ in non-alcoholic steatohepatitis (NASH) model mice. The result of a carrier administered group is in the left, the result of an administrated group with ADMPCs not treated with IL-1$\beta$ is in the middle, and the result of an administered group with ADMPCs treated with IL-1$\beta$ is in the right. The magnification on the top panels is 50 times, and the magnification on the bottom panels is 200 times.

Fig. 5 is a graph comparing reduction in liver tissue injury by ADMPCs treated with IL-1$\beta$ and ADMPCs not treated with IL-1$\beta$ in non-alcoholic steatohepatitis (NASH) model mice using NAFLD Activity Score. The p value is according to Mann-Whitney's U test.

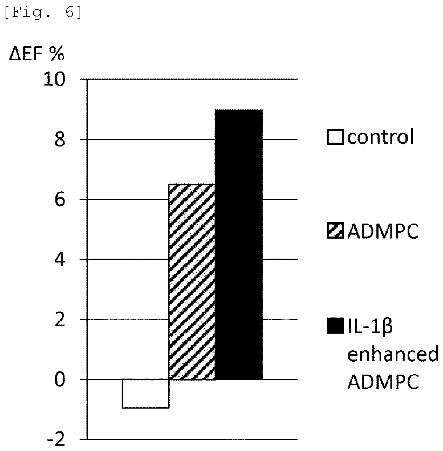

Fig. 6 is a graph illustrating improvement in left ventricular ejection fraction by ADMPCs treated with IL-1$\beta$ and ADMPCs not treated with IL-1$\beta$ in severe myocardial infarction model animals (pigs). The vertical axis ($\Delta$EF%) represents change (%) in left ventricular ejection fraction before and after administration of cells. The white bar represents a control group to which cells are not administered, the hatched bar represents a group to which ADMPCs not treated with IL-1$\beta$ are administered, and the black bar represents a group to which ADMPCs treated with IL-1$\beta$ are administered.

Mode for Carrying Out the Invention

In one aspect, the present invention provides a pharmaceutical composition for tissue healing, including adherent cells derived from mesenchymal tissue treated with a physiologically active polypeptide or LPS, and a pharmaceutically acceptable carrier. Here, the physiologically active polypeptide is a polypeptide that acts on a certain physiological regulatory function of the living body. Polypeptide refers to a substance in which two or more amino acid residues are linked to each other via a peptide bond. Various types of LPS are known.

The physiologically active polypeptides used in the present invention is a cytokine selected from interleukin-1 alpha or interleukin-1 beta. Other polypeptides are referred to and used in the examples of the present application for reference purposes, but are not according to the claimed invention. Such polypeptides further include one or more polypeptides selected from the group consisting of inflammatory cytokine, inflammatory cytokine-inducing polypeptide, growth factor, hormone and interferon. The inflammatory cytokine is a cytokine involved in pathogenesis of inflammation. The inflammatory cytokine-inducing polypeptide is a polypeptide having an effect of increasing the amount of inflammatory cytokine or enhancing the activity thereof. The growth factor is a polypeptide that promotes the growth or differentiation of specific cells in vivo. The chemokine is a basic protein that exhibits the action via a G protein coupled receptor and is a group of cytokines. The hormone is a substance that is produced in vivo, transported via body fluids, and affects the activity of specific cells, tissue or organ. The interferon is a group of cytokines produced in response to entry of foreign substances such as virus, pathogen or tumor cells in vivo. Various inflammatory cytokines, inflammatory cytokine-inducing polypeptides, growth factors and interferons are publicly known.

The cytokines include, but are not limited to, IL-1$\alpha$, IL-1$\beta$, IL-2 to IL-35, OSM (Oncostatin M), LIF, CNTF, CT-1, TNF-$\alpha$, TNF-$\beta$, BAFF, FasL, RANKL and TRAIL. The inflammatory cytokines include, but are not limited to, IL-1$\alpha$, IL-1$\beta$, IL-6, IL-8, IL-12, IL-18 and TNFa. Hereby only the use of **IL-1$\alpha$** and **IL-1$\beta$** is according to the claimed invention.

The inflammatory cytokine-inducing polypeptides include, but are not limited to, IL-17A.

The growth factors include, but are not limited to, activin A, ANGPTL5, BAFF, BD-2, BD-3, BNDF, BMP-1 to 7, DKK1, EGF, EG-VEGF, FGF-1 to 21, G-CSF, GM-CSF, HGF, IGF-1, IGF-2, platelet-derived growth factor (PDGF)-AA, PDGF-AB, PDGF-BB, R-spondin-1 to 3, SCF, galectin-1 to 3, GDF-11, GDNF, pleiotrophin, TGF-$\alpha$, TGF-$\beta$, TPO (thrombopoietin), TSLP, vascular endothelial growth factor (VEGF) and ciliary neurotrophic factor (CNTF).

The chemokines include, but are not limited to, CCL1 to CCL28 and CXCL1 to CXCL10.

The hormones include, but are not limited to, Calcitonin, Parathormone, Glucagon, Erythropoietin, Leptin, ANP, BNP, CNP, Oxytocin, Vasopressin, TRH (thyrotropin releasing hormone), TSH (thyroid stimulating hormone), CRH (corticotropin releasing hormone), ACTH (adrenocorticotropin hormone), GRH (gonadotropin releasing hormone), FSH (follicle stimulating hormone), LH (luteinizing hormone), SOM (somatostatin), GRH (growth hormone releasing hormone), GH (growth hormone), PRH (prolactin releasing hormone), PIH (prolactin inhibiting hormone) and Prolactin.

The interferons include, but are not limited to, IFN-$\alpha$, IFN-$\beta$, IFN-$\gamma$ and IFN-I.

The physiologically active peptides used in the present invention are **IL-1$\alpha$** and **IL-1$\beta$**.

The tissue healing refers to restoring a tissue to a normal state or bringing a tissue closer into a normal state, including tissue protection, repair of tissue/cell injury, promotion of proliferation of cells constituting a tissue, suppression of tissue inflammation, wound healing and reconstruction of the tissue form. Because the cells in the pharmaceutical composition of the present invention are useful for tissue protection, promotion of proliferation of cells constituting a tissue, etc., the pharmaceutical composition of the present invention is preferably used for tissue healing in chronic phase disease.

Tissues to be healed by the pharmaceutical composition of the present invention are any tissue of animal and are not particularly limited. Examples of the tissues include, but are not limited to, liver, pancreas, kidney, muscle, bone, cartilage, bone marrow, stomach, intestine, blood, nerve, skin, mucous membrane, heart and hair. Suitable tissues to be healed by the pharmaceutical composition of the present invention are liver, nerve, skin, mucous membrane and heart. Therefore, the pharmaceutical composition of the present invention is preferably used for treatment of, for example, liver cirrhosis, hepatitis and NASH (nonalcoholic steatohepatitis), and is also effective for chronic phase disease.

The cells that are an active ingredient of the pharmaceutical composition of the present invention are adherent cells derived from mesenchymal tissue treated with a physiologically active polypeptide or LPS.

The pharmaceutical composition of the present invention may be administered to a subject in the same species as or different species from the animal species from which the active ingredient cells are derived. For example, the pharmaceutical composition of the present invention including adherent cells derived from human-derived mesenchymal tissue treated with an inflammatory cytokine-inducing agent may be administered to a human subject. The cells in the pharmaceutical composition of the present invention may be from the same subject as the subject to be administered, or may be from a different subject from the subject to be administered.

The mesenchymal tissue-derived adherent cells used in the present invention may be commercially available ones, for example, distributed ones from organizations such as American Type Culture Collection (ATCC) (US) and NITE (Japan). Alternatively, mesenchymal tissue-derived adherent cells may be obtained from mesenchymal tissue. Means and methods for preparing mesenchymal tissue-derived adherent cells from mesenchymal tissue are publicly known.

Examples of mesenchymal tissue-derived adherent cells include mesenchymal tissue-derived stem cells (MSCs) such as adipose tissue-derived stem cells (ASCs), adipose tissue-derived multilineage progenitor cells (ADMPCs), Muse cells, cells derived from bone marrow tissue, umbilical cord tissue, amniotic tissue, cartilage tissue, periosteum tissue, synovium tissue, skeletal muscle tissue and placenta tissue, stem cells and stromal cells, and menstrual blood cells. The cells used according to the claimed invention are ADMPCs.

When cells are obtained from mesenchymal tissue, they may be isolated from any mesenchymal tissue. Examples of mesenchymal tissue include, but are not limited to, adipose tissue, bone marrow tissue, umbilical cord tissue, amniotic tissue, cartilage tissue, periosteum tissue, synovium tissue, skeletal muscle tissue, placenta tissue and menstrual blood. According to the claimed invention the cells are isolated from adipose tissue. This also is preferable because it is contained in a large amount in the body and many cells can be extracted.

Adherent cells can be obtained by extracting mesenchymal tissue from the body, placing and culturing the tissue in a culture vessel, and selectively acquiring cells adhering to the vessel. Mesenchymal tissue can be extracted using publicly known means and methods such as excision and aspiration. The extracted mesenchymal tissue may be cultured as it is, or if necessary, the extracted mesenchymal tissue may be minced or broken, followed by removing of red blood cells to culture the obtained cell population. These treating methods and means, and cell culturing means and methods are publicly known, and can be appropriately selected. Mesenchymal tissue-derived adherent cells may be obtained, for example, by treating the cells attached to the culture vessel with an enzyme such as trypsin.

Treatment of mesenchymal tissue-derived adherent cells with a physiologically active polypeptide or LPS may be carried out by contacting the cells with cytokine in a publicly known manner. Typically, this treatment may be carried out by culturing mesenchymal tissue-derived adherent cells for a certain period of time in a medium containing an appropriate concentration of physiologically active polypeptide or LPS. Usually, mesenchymal tissue-derived adherent cells are cultured in several nanograms/ml to several hundred nanograms/ml of inflammatory cytokine or a medium to which inflammatory cytokine has been added. The medium for use in culture may be a publicly known one. The culturing time and culturing temperature may also be appropriately selected. If necessary, mesenchymal tissue-derived adherent cells may be cultured to increase the number of cells, before treatment with a physiologically active polypeptide or LPS. A desired subpopulation may be obtained from a population of mesenchymal tissue-derived adherent cells, and if necessary, the subpopulation may be cultured to increase the number of cells, before treatment with a physiologically active polypeptide or LPS.

The number of types of physiologically active polypeptide or LPS for use in treatment of mesenchymal tissue-derived adherent cells may be one or two or more.

Mesenchymal tissue-derived adherent cells treated with a physiologically active polypeptide or LPS increases expression and production of one or more factors that contribute to tissue repair (such as polypeptide, growth factor and/or enzyme involved in tissue healing), or expresses and produces the same. Such factors include, but are not limited to, adiponectin, HGF, CSF2 (GM-CSF), CSF3 (G-CSF), LIF, MMP family factors, FGF family factors, ADAM family factors, angiopoietin-like protein family factors, pleiotrophin, R-spondin family factors and VEGF family factors. CSF2 or CSF3 contributes not only to activation of hematopoietic stem cells but also to stem cell proliferation and/or angiogenesis in many tissues or organs including brain, heart, lung and liver, thereby contributing to tissue repair. Accordingly, cells that express and produce these factors at higher level are preferred. The increase in expression or production of the above factor is according to the claimed invention 10 times or more, preferably 30 times or more, more preferably 50 times or more, still more preferably 100 times or more, compared to that before treatment.

The pharmaceutical composition of the present invention can be produced by mixing mesenchymal tissue-derived adherent cells treated with a physiologically active polypeptide or LPS as described above with a pharmaceutically acceptable carrier. A variety of pharmaceutically acceptable carriers are publicly known and may be appropriately selected for use. For example, when the pharmaceutical composition of the present invention is used as an injection, the cells may be suspended in a carrier such as purified water, saline or phosphate buffered saline.

The dosage form of the pharmaceutical composition of the present invention is not particularly limited, but may be a solution, semisolid or solid. The administration method of the pharmaceutical composition of the present invention is also not limited, but may include local injection, intravenous injection or infusion, application to an affected area, administration to an affected area via a catheter, or direct transplantation to tissues such as liver by a surgical procedure. The pharmaceutical composition of the present invention may be transplanted in the form of cell sheet, cell mass, layered cell sheet, etc.

The administration route and dose of the pharmaceutical composition of the present invention may be appropriately determined in consideration of the type and site of the tissue to be healed, the degree of disease, the condition of the subject and the like.

The pharmaceutical composition of the present invention may contain cells other than adherent cells derived from mesenchymal tissue treated with a physiologically active polypeptide or LPS.

In a further aspect, the present invention provides use of adherent cells derived from mesenchymal tissue treated with a physiologically active polypeptide or LPS in producing a medicament for tissue healing.

In a further aspect, the present invention provides the adherent cells derived from mesenchymal tissue treated with a physiologically active polypeptide or LPS for use in tissue healing.

In yet another aspect, the present invention provides a method for producing a pharmaceutical composition for tissue healing, including the steps of:

(a) treating adherent cells derived from mesenchymal tissue with a physiologically active polypeptide or LPS, and

(b) mixing the cells treated in step (a) with a pharmaceutically acceptable carrier.

In yet another aspect, the present invention provides a method for producing cells for tissue healing, including treating adherent cells derived from mesenchymal tissue with a physiologically active polypeptide or LPS.

Hereinafter, more detailed and specific description is made of the present invention with reference to Examples, but the Examples are not intended to limit the present invention.

Example 1

Example 1. Effect of treating ADMPCs with IL-Iβ

(1) Method of experiment

(i) Collection of adipose tissue from human subject

From six women from which informed consent was obtained, extra adipose tissue to be discarded was received during liposuction surgery. The protocol conformed to the Kobe University Graduate School of Medicine Review Boards for Human Research.'

(ii) Isolation and culture of ADMPCs

The adipose tissue was minced and then digested in Hanks' buffered saline solution (HBSS) containing 0.008% Liberase (Roche Lifescience) with shaking in a water bath at 37°C for 1 hour. The digested product was filtered through Cell Strainer (BD Bioscience), followed by centrifuging at $800 \times g$ for 10 minutes. The lymphocyte separation solution (d = 1.077) (Nacalai tesque) was used to remove red blood cells by specific gravity method. Cells in the obtained cell population containing ADMPCs were seeded in DMEM containing 10% fetal bovine serum (Hyclone) to allow for attachment of the cells, followed by washing and treatment with EDTA to yield ADMPCs. Then, the ADMPCs in a medium (60% DMEM-low glucose, 40% MCDB201, 10 ug/mL EGF, 1 nM dexamethasone, 100 μM ascorbic acid and 5% FBS) were seeded on a human fibronectin-coated dish and subcultured 3 to 8 times to yield cultured ADMPCs.

(iii) IL-1β treatment

IL-1β was added to a medium (60% DMEM-low glucose, 40% MCDB 201, 10 μg/mL EGF, 1 nM dexamethasone, 100 μM ascorbic acid and 5% FBS) to a concentration of 10 ng/ml. The cultured ADMPCs obtained in (ii) above were cultured in the IL-1β-containing medium for 72 hours to measure adiponectin and hepatocyte growth factor (HGF) produced in the medium. The measurement of adiponectin was performed using the ELISA kit of abcam (Catalog No. ab99968). The measurement of HGF was performed using the ELISA kit of R & D System (Catalog No. DHG00). For a control, ADMPCs were cultured in the above medium except than IL-1β was not added.

(2) Results of experiment

The measurement results of amount of adiponectin produced are shown in Fig. 1. Production of adiponectin from ADMPCs not treated with IL-1β was not found, but it was confirmed that adiponectin was produced from ADMPCs treated with IL-1β.

The measurement results of amount of HGF produced are shown in Fig. 2. The amount of HGF produced from ADMPCs treated with IL-1β was increased by about 1.7 times as compared to the amount of HGF produced from ADMPCs not treated with IL-1β.

Example 2

Example 2. In vivo tissue healing effect of ADMPCs treated with IL-1β - alleviation of liver tissue injury

(1) Method of experiment

Collection of adipose tissue from a human subject, isolation and culture of ADMPCs, and IL-1β treatment were performed in the same manner as in Example 1, except that the concentration of IL-1B in the medium was 5 ng/ml.

ADMPCs treated with IL-1β were suspended in a carrier to a concentration of $1.2 \times 10^5$ cells/ml. This was administered to NASH model mice (STAM (registered trademark) mice) to examine healing of the liver tissue. The animals were divided into 3 groups: administrated group with ADMPCs treated with IL-1β (n = 9), administrated group with ADMPCs not treated with IL-1β (n = 9), and carrier administered group (n = 10)). At the beginning of the study, animals in each group were intradermally administered with streptozotocin, fed with a normal diet, fed with a high-fat diet from week 4 to week 9, and euthanized at week 9. Administration of ADMPCs ($3 \times 10^5$ cells/kg) or carrier was performed once at week 6. The liver tissue sections obtained were subjected to Sirius red staining and hematoxylin-eosin (HE) staining.

(2) Results of experiment

(i) Sirius red staining of liver tissue sections

The results of Sirius red staining of liver tissue sections from mice obtained at week 9 of the study are shown in Fig. 3. In the livers from mice administered with ADMPCs treated with IL-1β, it was confirmed that deposition of intrahepatic fibers stained with Sirius red was reduced, compared to the livers from carrier administered mice and mice administered with ADMPCs not treated with IL-1β.

(ii) HE staining of liver tissue sections

The results of HE staining of liver tissue sections from mice obtained at week 9 of the study are shown in Fig. 4. In the livers from mice administered with ADMPCs treated with IL-1β, it was confirmed that injury to liver tissue represented by vacuolation was reduced, compared to the livers from carrier administered mice and mice administered with ADMPCs not treated with IL-1β.

(iii) Evaluation of liver tissue healing effect by NAFLED activity score (E. M. Brunt et al. Hepatology. 2011 March; 53(3): 810-820)

The degree of liver tissue injury in mice obtained at week 9 of the study was evaluated according to the NAFLED activity score. The evaluation method of NAFLED activity score is shown in Table 1.

[Table 1]

EVALUATION METHOD OF NAFLD ACTIVITY SCORE

| ITEM | DEFINITION | SCORE |
|---|---|---|
| FATTY CHANGE | FATTY CHANGE AT LOW TO MODERATE MAGNIFICATION | |
| | < 5% | 0 |
| | 5-33% | 1 |
| | 33-66° | 2 |
| | > 66% | 3 |
| INFLAMMATION OF LIVER PARENCHYMA | EVALUATION OF INFLAMMATION FOCI | |
| | NONE | 0 |
| | LESS THAN 2 SITES AT 200 TIMES ENLARGEMENT | 1 |
| | 2 TO 4 SITES AT 200 TIMES ENLARGEMENT | 2 |
| | MORE THAN 5 SITES AT 200 TIMES ENLARGEMENT | 3 |
| LIVER CELL INJURY (BALLOONING) | NONE | 0 |
| | 2 TO 3 BALLOONING CELLS | 1 |
| | 4 OR MORE BALLOONING CELLS | 2 |

The results are shown in Fig. 5. Because the NAFLD activity score of the livers from mice administered with ADMPCs treated with IL-1β was significantly lower, compared to the livers from carrier administered mice and mice administered with ADMPCs not treated with IL-1β, it was confirmed that injury to liver tissue represented by vacuolation, inflammation and fatty change was greatly reduced.

From these results, ADMPCs treated with IL-1β are found to be effective in healing injured tissue, and be useful for tissue protection, repair of tissue/cell injury, suppression of tissue inflammation and promotion of proliferation of cells constituting a tissue. These tissue healings are considered to allow for reconstruction of the tissue form and wound healing. Moreover, because these effects were observed in mice in which liver injury was induced by streptozotocin and high-fat diet, it can be said that IL-1β-treated ADMPCs are effective for tissue healing in chronic phase disease.

Example 3

Example 3. In vivo tissue healing effect of ADMPCs treated with IL-1β - improvement of cardiac function

(1) Method of experiment

A severe myocardial infarction model was created using 8 weeks old pigs by two-stage embolism/reperfusion method. Specifically, a 6F guide catheter was transcutaneously placed through the femoral artery on the opening of the left coronary artery, a guide wire was inserted through the catheter into the first diagonal artery (# 9 in the AHA classification), and preconditioning was performed by conducting ballooning (obstruction reopening) with the aid of the guide. One week later, a guidewire was inserted into the left anterior descending coronary artery (# 6 to # 8 in the AHA classification), and ballooning (obstruction reopening) was performed at the left anterior descending coronary artery immediately below the bifurcation of the left circumflex coronary artery (# 6 in the AHA classification) to produce a myocardial ischemic region. Four weeks after that (five weeks after the first obstruction reopening), individuals with a cardiac ejection fraction of 40% or less in cardiac ultrasonography were subjected to the study as a severe heart failure model.

Four weeks after the second embolism/reperfusion, the animals were divided into 3 groups: a control (cells were not administrated) group, group to which non-activated cells (ADMPCs) were administered at a concentration of $3 \times 10^5$ cells/kg body weight through a catheter via the coronary artery, and group to which IL-1β activated cells (72 hours cultured) (IL-1β-activated ADMPCs) were administered in the same manner. Preparation of ADMPCs and IL-1β-activated ADMPCs was carried out in the same manner as in Example 1. Immediately before administration, cardiac MRI was performed 3 months after administration (Signa EXCITE XI TwinSpeed 1.5T Ver. 11.1, GE Healthcare), using Cardiac Vx (GE Healthcare) as analysis software, to measure left ventricular end-diastolic and end-systolic volumes. The Formula:

```
Left ventricular ejection fraction = 100 × (left ventricular
end-diastolic  volume  -  left  ventricular  end-systolic
volume)/(left ventricular end-diastolic volume)
```

was used to calculate left ventricular ejection fraction (% EF) to represent the difference between the value 3 months after administration and the value immediately before administration as ΔEF (%) (Fig. 6).

(2) Results of experiment

As shown in Fig. 6, the left ventricular ejection fraction was decreased in the control group, whereas the left ventricular ejection fraction was improved in the two groups to which cells were administered, in particular, when IL-1β-activated cells were administered, the left ventricular ejection fraction was markedly improved.

These results indicate that IL-1β-treated ADMPCs heal cardiac tissue injured by severe myocardial infarction and markedly improve the cardiac function.

Example 4

Example 4. Effect of treating adhesive cells derived from various mesenchymal tissues with various physiologically active polypeptides

Only the examples with ADMPCs as the test cells, in combination with **IL-1α** and **IL-1β** as the physiologically active peptides, are according to the claimed invention. The examples with other types of test cells and/or other types of physiologically active polypeptides are to be considered as reference-examples.

(1) Method of experiment

As test cells, umbilical cord-derived mesenchymal stem cells (umbilical cord-derived MSCs), adipose tissue-derived stem cells (ADSCs), knee cartilage synovium-derived mesenchymal stem cells (synovium-derived MSCs), adipose tissue-derived multilineage progenitor cells (ADMPCs), placenta-derived mesenchymal stem cells (placenta-derived MSCs) and bone marrow-derived mesenchymal stem cells (bone marrow-derived MSCs) were used. Various cytokines, chemokines, growth factors and hormones were used as physiologically active polypeptides.

When ADMPCs were used as test cells, cytokines (IL-1$\alpha$, IL-1$\beta$, IL3 to IL35, oncostatin M, LIF, CNTF, CT-1, TNF$\alpha$, TNF$\beta$, BAFF, FasL, RANKL, TRAIL, INF-$\alpha$, IFN-$\beta$, IFN-$\gamma$), chemokines (CCL1 to CCL28, CXCL1 to CXCL10), growth factors (AvinA, ANGFLT5, BD-2, BD-3, BDNF, BMP-1 to BMP-7, DKK1, EGF, EG-VEGF, FGF-1 to FGF-21, G-CSF, HGF, IGF-1, IGF-2, PDGF-AA, PDGF-BB, R-spondin-1, R-spondin-2, R-spondin-3, SCF, galectin 1, galectin 2, galectin 3, GDF-11, GDNF, pleiotrophin, TGF$\alpha$, TGF$\beta$, TPO, TSLP, VEGF), and hormones (calcitonin, parathormone, glucagon, erythropoietin, leptin, ANP, BNP, CNP, oxytocin, vasopressin, TGH, TSH, CRH, ACTH, GRH, FSH, LH, SOM, GRH, GH, PRH, prolactin) were used as physiologically active polypeptides. When ADSCs, placenta-derived MSCs, synovium-derived MSCs, bone marrow-derived MSCs and umbilical cord-derived MSCs are used as test cells, IL-1$\alpha$, IL-1$\beta$, TNF$\alpha$, TNF$\beta$, IFN-$\beta$, IFN-$\gamma$, FGF15 are used as physiologically active polypeptides. Hereinafter, the physiologically active polypeptide is referred to as "drug". The test cells were subjected to medium replacement with a drug-containing medium (final concentration of 100 ng/mL) and drug-free medium (control), and further subcultured for 3 days (72 hours). After 72 hours of medium change, 600 $\mu$L of RLT Buffer was added for recovery and RNA extraction.

As to RLT Buffer samples, total RNA was extracted using RNeasy Mini Kit/QIAGEN, and the total RNA was prepared in a concentration of 100 ng/uL. Then, labeled cRNA was synthesized from 150 ng of the total RNA per array. For the synthesized labeled cRNA, the concentration, yield and Cy3 uptake rate were calculated and the synthetic size (200 to 2000 nt were amplified) was measured. Six hundred ng of the labeled cRNA was fragmented at 60°C and hybridized at 65°C for 17 hours, and the array was washed and scanned.

A probe with the measured value reliable was extracted under the condition of either the control sample or the drug-added sample (one type), and the probe having an expression difference of 15 times or more was extracted as compared to the control sample.

(2) Results of experiment

Tables 2 to 7 show mRNAs whose expression was increased by 15 times or more after treatment with the drug as compared to those before treatment, and their multiplication factor.

[Table 2]

| UMBILICAL CORD-DERIVED MSCs | | |
|---|---|---|
| DRUG | INCREASED GENE | MULTIPLICATION FACTOR |
| TNF$\alpha$ | CSF2 | 51.92866 |
| IFN$\gamma$ | SPARCL1 | 24.166359 |
| IL1$\alpha$ | CSF2 | 65.41428 |
| | CCL3 | 44.713257 |
| | MMP3 | 17.676903 |
| IL1$\beta$ | CSF2 | 45.73727 |
| FGF15 | MMP7 | 20.78586 |

[Table 3]

| ADSCs | | |
|---|---|---|
| DRUG | INCREASED GENE | MULTIPLICATION FACTOR |
| TNF-α | CSF3 | 457.4336 |
| | CSF2 | 210.81802 |
| | MMP9 | 36.619884 |
| | LIF | 28.586313 |
| | FGF13 | 26.435118 |
| | BMP2 | 24.754152 |
| | MMP3 | 16.642172 |
| TNF-β | MMP9 | 50.442356 |
| | CSF3 | 23.138222 |
| | FGF5 | 15.041612 |
| IFNβ | CSF3 | 81.64009 |
| | CSF2 | 81.4202 |
| | BTC | 37.966434 |
| | FGF20 | 33.667175 |
| IFNγ | FGF20 | 17.39512 |
| | MMP25 | 15.073852 |
| IL-1α | CSF2 | 3650.884 |
| | CSF3 | 3004.3464 |
| | MMP3 | 153.17429 |
| | MMP12 | 48.928066 |
| | LIF | 44.622696 |
| | NTN1 | 19.101036 |
| | HBEGF | 17.43808 |
| | MMP1 | 16.788137 |
| | MMP8 | 15.74857 |
| IL-1β | CSF3 | 3658.2717 |
| | CSF2 | 2945.6265 |
| | MMP3 | 163.10434 |
| | MMP12 | 77.35472 |
| | LIF | 44.191887 |
| | MMP8 | 20.121588 |
| | HBEGF | 17.731503 |
| | MMP1 | 17.499699 |
| | ADAMTSS | 16.701633 |
| | FGF13 | 15.317384 |

(continued)

| ADSCs | | |
|---|---|---|
| DRUG | INCREASED GENE | MULTIPLICATION FACTOR |
| FGF15 | CSF3 | 1992.8231 |
| | CSF2 | 177.31819 |
| | MMP3 | 87.44734 |
| | MMP12 | 18.993986 |

[Table 4]

| SYNOVIUM-DERIVED MSCs | | |
|---|---|---|
| DRUG | INCREASED | MULTIPLICATION . |
| TNF$\alpha$ | CSF3 | 481.9497 |
| | MMP3 | 184.5146 |
| | MMP1 | 118.73493 |
| | CSF2 | 93.68834 |
| | RSPO3 | 78.78901 |
| | RSPO3 | 54.598293 |
| | MMP12 | 37.193733 |
| | ANGPTL1 | 29.710234 |
| | LIF | 19.802446 |
| TNF-$\beta$ | CSF3 | 133.98564 |
| | MMP3 | 79.96354 |
| | MMP1 | 57.18347 |
| | RSPO3 | 29.265568 |
| | RSPO3 | 22.514166 |
| IFN$\beta$ | ANGPTL1 | 30.57255 |
| | FGF20 | 17.808767 |
| IFN-$\gamma$ | MMP2 5 | 18.338886 |
| IL-1$\alpha$ | CSF3 | 10575.728 |
| | MMP3 | 1345.4216 |
| | MMP1 | 244.72272 |
| | MMP12 | 163.36778 |
| | CSF2 | 142.88773 |
| | MMP13 | 28.037321 |
| | MMP10 | 21.2812 |
| | RSPO3 | 18.53049 |

(continued)

| SYNOVIUM-DERIVED MSCs | | |
|---|---|---|
| DRUG | INCREASED | MULTIPLICATION . |
| IL1β | CSF3 | 8791.783 |
| | MMP3 | 935.04913 |
| | MMP12 | 181.83107 |
| | CSF2 | 129.32849 |
| | MMP1 | 107.597824 |
| | ADAMTS16 | 33.420284 |
| | GDF3 | 16.958546 |
| | MMP13 | 16.034931 |
| | IGF1 | 15.101902 |
| FGF15 | CSF3 | 1407.7273 |
| | MMP3 | 490.81107 |
| | MMP12 | 162.75064 |
| | MMP1 | 58.44772 |
| | IGF1 | 58.422787 |
| | BMP6 ! | 20.44636 |

[Table 5-1]

| ADMPCs | | |
|---|---|---|
| DRUG | INCREASED GENE | MULTIPLICATION FACTOR |
| BMP-3 | GCG | 17.480957 |
| | NRTN | 28.843102 |
| BMP-4 | REG4 | 75.89187 |
| BMP-6 | HDGFL1 | 65.01833 |
| CCL-3 | EGF | 15.73962 |
| CCL-5 | MMP2 6 | 29.093975 |
| | MMP13 | 17.447323 |
| CCL-8 | BMP7 | 17.2787 |
| CCL-9 | BMP10 | 28.345194 |
| CCL-15 | BMP7 | 17.304893 |
| CCL-19 | FGF22 | 47.299706 |
| CCL-20 | EGF | 34.020695 |
| CCL-21 | BMP10 | 30.952303 |
| CCL-23 | ADIPOQ | 18.862783 |
| CCL-26 | FGF10 | 15.473124 |
| CCL-28 | NMU | 22.596947 |

(continued)

| ADMPCs | | |
|---|---|---|
| DRUG | INCREASED GENE | MULTIPLICATION FACTOR |
| CNTF | ADAMTS20 | 44.9686 |
| | EGF | 20.999825 |
| CT-1 | FGF6 | 16.76011 |
| CXCL5 | ADAM22 | 15.880237 |
| CXCL10 | ADIPOQ | 23.21833 |
| | LTBP3 | 15.590775 |
| FGF15 | CSF3 | 78.26141 |
| | MMP3 | 21.577183 |
| GALECTIN 1 | IGF1 | 17.737694 |
| IFNβ | PTN | 34.09604 |
| | ANGPTL1 | 23.657429 |
| IFNγ | ADAMTSS | 18.498682 |
| | GLDN | 93.92384 |
| | PGF | 33.75676 |
| | FGF21 | 16.46351 |
| IGF-2 | IGFL4 | 18.903763 |
| IL-1α | CSF3 | 1266.3649 |
| | CSF2 | 241.81323 |
| | MMP8 | 90.97638 |
| | MMP3 | 82.76877 |
| | MMP12 | 62.105858 |
| | FGF13 | 61.590416 |
| | MMP1 | 30.74703 |
| | MMP13 | 21.096134 |

[Table 5-2]

| DRUG | INCREASED GENE | MULTIPLICATION FACTOR |
|---|---|---|
| IL-1β | CSF3 | 1414.8679 |
| | CSF2 | 215.49258 |
| | MMP8 | 82.693 |
| | MMP3 | 81.932846 |
| | MMP12 | 80.88991 |
| | FGF13 | 53.733124 |
| | MMP1 | 29.282549 |
| | MMP13 | 19.679766 |
| IL-7 | BMP3 | 15.619323 |

14

(continued)

| DRUG | INCREASED GENE | MULTIPLICATION FACTOR |
|---|---|---|
| IL-8 | EGF | 166.93967 |
| | ADAMTS20 | 27.746012 |
| | MMP7 | 15.016879 |
| IL-11 | OOSP2 | 27.152683 |
| IL-17 | CSF3 | 47.33713 |
| IL-24 | FGF20 | 16.994074 |
| IL-26 | MMP26 | 25.349789 |
| IL-31 | ADAM21 | 16.247 |
| IL-35 | NPPC | 27.392384 |
| | EPO | 21.361319 |
| TNFα | CSF3 | 760.7263 |
| | FGF13 | 519.2593 |
| | MMP8 | 322.53482 |
| | CSF2 | 84.63178 |
| | MMP3 | 42.06483 |
| | MMP1 | 41.600243 |
| | MMP13 | 33.378723 |
| | MMP10 | 33.15327 |
| | FGL2 | 30.280602 |
| | ADAM8 | 28.907818 |
| | MMP9 | 24.30117 |
| | RSFC3 | 22.690266 |
| TNFβ | FGF13 | 52.990948 |
| | CSF2 | 27.081493 |
| | CSF3 | 25.747252 |
| | MMP8 | 23.552876 |
| | MMP9 | 18.909662 |
| | MMP1 | 18.20611 |
| | MMP3 | 15.417225 |
| TRAIL | OOSP2 | 30.486296 |

[Table 6]

| PLACENTA-DERIVED MSCs | | |
|---|---|---|
| DRUG | INCREASED GENE | MULTIPLICATION FACTOR |
| TNFα | CSF2 | 359.60034 |
| | MMP1 | 157.07306 |
| | FGF13 | 138.73257 |
| | CSF3 | 52.898098 |
| | MMP9 | 52.12447 |
| | RSPO3 | 36.97124 |
| | MMP12 | 28.326378 |
| | LIF | 20.185322 |
| TNFβ | CSF2 | 116.80491 |
| | MMP1 | 35.85062 |
| | RSPO3 | 33.528667 |
| | FGF13 | 30.268114 |
| IFNβ | ANGPTL1 | 67.8647 |
| IL1α | CSF3 | 4009.4556 |
| | CSF2 | 925.7604 |
| | MMP1 | 86.76511 |
| | MMP12 | 44.755383 |
| | EGF | 32.053967 |
| | MMP3 | 30.25197 |
| | MMP20 | 19.222319 |
| IL1β | CSF2 | 2408.85 |
| | CSF3 | 6519.517 |
| | EGF | 24.028 |
| | FGF13 | 18.65617 |
| | MMP1 | 143.90538 |
| | MMP12 | 87.97726 |
| | MMP3 | 50.21765 |
| FGF15 | CSF3 | 653.6217 |
| | CSF2 | 235.56853 |
| | MMP1 | 16.586615 |

[Table 7]

| BONE MARROW-DERIVED MSCs | | |
|---|---|---|
| DRUG | INCREASED GENE | MULTIPLICATION FACTOR |
| TNFα | MMP1 | 62.074005 |
| | MMP3 | 39.413765 |
| | CSF2 | 34.723072 |
| | RSPO3 | 20.590866 |
| TNFβ | MMP1 | 16.974045 |
| IFNβ | BTC | 23.89296 |
| | DLL1 | 20.705479 |
| | PTN | 16.424856 |
| IFNγ | ANGPTL5 | 98.50543 |
| | RSPO3 | 17.248734 |
| IL1α | MMP3 | 545.55664 |
| | CSF2 | 248.18904 |
| | MMP12 | 81.264694 |
| | MMP1 | 20.65767 |
| | CSF3 | 17.709955 |
| IL1β | CSF2 | 544.1642 |
| | MMP3 | 482.9409 |
| | MMP12 | 55.837635 |
| | NTN1 | 34.482384 |
| | MMP1 | 28.635344 |
| | CSF3 | 17.319433 |
| FGF15 | MMP3 | 46.422943 |
| | MMP1 | 21.592436 |

In any of the experiments, it was confirmed that expression of polypeptides, growth factors and/or enzymes involved in tissue healing was enhanced in adherent cells derived from mesenchymal tissue treated with a physiologically active polypeptide. In many combinations of the physiologically active polypeptide and adherent cells derived from mesenchymal tissue, CSF2 and/or CSF3 tended to be expressed, in particular highly expressed.

Industrial Applicability

The present invention is useful in the field of medicines for tissue healing and in the field of research of diseases requiring tissue healing.

Claims

1. A method for producing a pharmaceutical composition for tissue healing in chronic phase disease, comprising the steps of:

(a) treating adipose tissue-derived multilineage progenitor cells (ADMPCs)with interleukin-1 alpha (IL-1α) or interleukin-1 beta (IL-1β); and
(b) mixing the cells treated in step (a) with a pharmaceutically acceptable carrier, wherein ADMPCs treated with interleukin-1 alpha (IL-1α) or interleukin-1 beta (IL-1β) increase expression and production of one or more factors that contribute to tissue repair selected from the group consisting of adiponectin, HGF, CSF2 (GM-CSF), CSF3 (G-CSF), MMP-1, MMP-3, MMP-8, MMP-12, MMP-13 and FGF-13, and wherein the increase in expression or production of the one or more factors that contribute to tissue repair is 10 times or more after the treatment compared to that before treatment

2. The method according to claim 1, wherein the one or more factors that contribute to tissue repair are selected from the group consisting of CSF2 and CSF3.

3. The method according to any one of claims 1 to 2, wherein the tissue healing is tissue protection, repair of tissue/cell injury, promotion of proliferation of cells constituting a tissue, suppression of tissue inflammation, wound healing or reconstruction of tissue form.

4. The method according to any one of claims 1 to 3, wherein the tissue is liver tissue or cardiac tissue.

5. A pharmaceutical composition for tissue healing obtainable by a method according to any one of claims 1 to 4, 11 Lv , comprising adipose tissue-derived multilineage progenitor cells (ADMPCs)treated with interleukin-1 alpha (IL-1α) or interleukin-1 beta (IL-1β), wherein said treated ADMPCs increase expression and production of one or more factors that contribute to tissue repair selected from the group consisting of adiponectin, HGF, CSF2 (GM-CSF), CSF3 (G-CSF), MMP-1, MMP-3, MMP-8, MMP-12, MMP-13 and FGF-13, and wherein the increase in expression or production of the one or more factors that contribute to tissue repair is 10 times or more after the treatment compared to that before treatment,
and a pharmaceutically acceptable carrier.

6. The pharmaceutical composition of claim 5, wherein the one or more factors that contribute to tissue repair are selected from the group consisting of CSF2 and CSF3.

7. A pharmaceutical composition of any of claims 5 or 6 for use in tissue healing in chronic phase disease.

8. The pharmaceutical composition for use according to claim 7, wherein the tissue healing is tissue protection, repair of tissue/cell injury, promotion of proliferation of cells constituting a tissue, suppression of tissue inflammation, wound healing or reconstruction of tissue form.

9. The pharmaceutical composition for use according to any one of claims 7 or 8, wherein the tissue is liver tissue or cardiac tissue.

**Patentansprüche**

1. Ein Verfahren zur Herstellung einer pharmazeutischen Zusammensetzung zur Gewebeheilung in der chronischen Phase einer Krankheit, umfassend die Schritte:

(a) Behandeln von aus Fettgewebe gewonnenen multilinearen Vorläuferzellen (ADMPCs) mit Interleukin-1 alpha (IL-1α) oder Interleukin-1 beta (IL-1β); und
(b) Mischen der in Schritt (a) behandelten Zellen mit einem pharmazeutisch verträglichen Träger, wobei die mit Interleukin-1 alpha (IL-1α) oder Interleukin-1 beta (IL-1β) behandelten ADMPCs die Expression und Produktion eines oder mehrerer Faktoren erhöhen, die zur Gewebereparatur beitragen, ausgewählt aus der Gruppe bestehend aus Adiponectin, HGF, CSF2 (GM-CSF), CSF3 (G-CSF), MMP-1, MMP-3, MMP-8, MMP-12, MMP-13 und FGF-13, und wobei die Erhöhung der Expression oder Produktion des einen oder der mehreren Faktoren, die zur Gewebereparatur beitragen, nach der Behandlung im Vergleich zu derjenigen vor der Behandlung das 10-fache oder mehr beträgt.

2. Das Verfahren nach Anspruch 1, wobei der eine oder die mehreren Faktoren, die zur Gewebereparatur beitragen, ausgewählt sind aus der Gruppe bestehend aus CSF2 und CSF3.

3. Das Verfahren nach einem der Ansprüche 1 bis 2, wobei es sich bei der Gewebeheilung um Gewebeschutz, Reparatur einer Gewebe-/Zellverletzung, Förderung der Proliferation von Zellen, die ein Gewebe bilden, Unterdrückung von Gewebeentzündungen, Wundheilung oder Wiederherstellung der Gewebeform handelt.

**4.** Das Verfahren nach einem der Ansprüche 1 bis 3, wobei es sich bei dem Gewebe um Lebergewebe oder Herzgewebe handelt.

**5.** Eine pharmazeutische Zusammensetzung zur Gewebeheilung, erhältlich durch ein Verfahren nach einem der Ansprüche 1 bis 4, umfassend

aus Fettgewebe gewonnene multilineare Vorläuferzellen (ADMPCs), die mit Interleukin-1 alpha (IL-1$\alpha$) oder Interleukin-1 beta (IL-1$\beta$) behandelt wurden, wobei die behandelten ADMPCs die Expression und Produktion eines oder mehrerer Faktoren erhöhen, die zur Gewebereparatur beitragen, ausgewählt aus der Gruppe bestehend aus Adiponectin, HGF, CSF2 (GM-CSF), CSF3 (G-CSF), MMP-1, MMP-3, MMP-8, MMP-12, MMP-13 und FGF-13, und wobei die Erhöhung der Expression oder Produktion des einen oder der mehreren Faktoren, die zur Gewebereparatur beitragen, nach der Behandlung im Vergleich zu derjenigen vor der Behandlung das 10-fache oder mehr beträgt, und einen pharmazeutisch verträglichen Träger.

**6.** Die pharmazeutische Zusammensetzung nach Anspruch 5, wobei der eine oder die mehreren Faktoren, die zur Gewebereparatur beitragen, ausgewählt sind aus der Gruppe bestehend aus CSF2 und CSF3.

**7.** Eine pharmazeutische Zusammensetzung nach einem der Ansprüche 5 oder 6 zur Verwendung bei der Gewebeheilung in der chronischen Phase einer Krankheit.

**8.** Die pharmazeutische Zusammensetzung zur Verwendung nach Anspruch 7, wobei es sich bei der Gewebeheilung um Gewebeschutz, Reparatur einer Gewebe-/Zellverletzung, Förderung der Proliferation von Zellen, die ein Gewebe bilden, Unterdrückung von Gewebeentzündungen, Wundheilung oder Wiederherstellung der Gewebeform handelt.

**9.** Die pharmazeutische Zusammensetzung zur Verwendung nach einem der Ansprüche 7 oder 8, wobei es sich bei dem Gewebe um Lebergewebe oder Herzgewebe handelt.

**Revendications**

**1.** Procédé de production d'une composition pharmaceutique pour la cicatrisation des tissus dans une maladie en phase chronique, comprenant les étapes consistant à :

(a) traiter des cellules progénitrices multilignées dérivées du tissu adipeux (ADMPC) avec l'interleukine-1 alpha (IL-1$\alpha$) ou l'interleukine-1 beta (IL-1$\beta$) ; et
(b) mélanger les cellules traitées dans l'étape (a) avec un support pharmaceutiquement acceptable, où les cellules ADMPC traitées avec l'interleukine-1 alpha (IL-1$\alpha$) ou l'interleukine-1 beta (IL-1$\beta$) augmentent l'expression et la production d'un ou plusieurs facteurs qui contribuent à la réparation du tissu sélectionnés parmi le groupe constitué de l'adiponectine, HGF, CSF2 (GM-CSF), CSF3 (G-CSF), MMP-1, MMP-3, MMP-8, MMP-12, MMP-13, et FGF-13, et où l'augmentation de l'expression ou de la production des un ou plusieurs facteurs qui contribuent à la réparation du tissu est 10 fois ou plus après le traitement par rapport à ce qu'elle était avant le traitement.

**2.** Procédé selon la revendication 1, dans lequel les un ou plusieurs facteurs qui contribuent à la réparation du tissu sont sélectionnés parmi le groupe constitué de CSF2 et CSF3.

**3.** Procédé selon l'une quelconque des revendications 1 à 2, dans lequel la cicatrisation du tissu est la protection du tissu, la réparation d'une lésion tissulaire/cellulaire, la promotion de la prolifération des cellules constituant un tissu, la suppression d'une inflammation tissulaire, la cicatrisation d'une plaie ou la reconstruction de la forme tissulaire.

**4.** Procédé selon l'une quelconque des revendications 1 à 3, dans lequel le tissu est du tissu hépatique ou du tissu cardiaque.

**5.** Composition pharmaceutique pour la cicatrisation des tissus pouvant être obtenue par un procédé selon l'une quelconque des revendications 1 à 4, comprenant des cellules progénitrices multilignées dérivées du tissu adipeux (ADMPC) traitées avec l'interleukine-1 alpha (IL-1$\alpha$) ou l'interleukine-1 beta (IL-1$\beta$), où lesdites cellules ADMPC traitées augmentent l'expression et la production d'un ou plusieurs facteurs qui contribuent à la réparation du tissu sélectionnés parmi le groupe constitué de l'adiponectine, HGF, CSF2 (GM-CSF), CSF3 (G-CSF), MMP-1, MMP-3, MMP-8, MMP-12, MMP-13, et FGF-13, et où l'augmentation de l'expression ou de la production des un ou plusieurs facteurs qui contribuent à la réparation du tissu est 10 fois ou plus après le traitement par rapport à ce qu'elle était avant le traitement,

et un support pharmaceutiquement acceptable,

6. Composition pharmaceutique selon la revendication 5, dans laquelle les un ou plusieurs facteurs qui contribuent à la réparation du tissu sont sélectionnés parmi le groupe constitué de CSF2 et CSF3.

7. Composition pharmaceutique selon l'une quelconque des revendications 5 ou 6, à utiliser dans la cicatrisation des tissus dans une maladie en phase chronique,

8. Composition pharmaceutique à utiliser selon la revendication 7, dans laquelle la cicatrisation du tissu est la protection du tissu, la réparation d'une lésion tissulaire/cellulaire, la promotion de la prolifération des cellules constituant un tissu, la suppression d'une inflammation tissulaire, la cicatrisation d'une plaie, ou la reconstruction de la forme tissulaire,

9. Composition pharmaceutique à utiliser selon l'une quelconque des revendications 7 ou 8, dans laquelle le tissu est du tissu hépatique ou du tissu cardiaque.

[Fig. 1]

ADIPONECTIN

[Fig. 2]

HGF

[Fig. 3]

CARRIER    hADMPC    IL-1β-TREATED hADMPC

[Fig. 4]

CARRIER  hADMPC  IL-1β-TREATED hADMPC

[Fig. 5]

[Fig. 6]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008153179 A

- WO 2009154770 A

**Non-patent literature cited in the description**

- **ZUK PA ; ZHU M ; ASHJIAN P et al.** Human adipose tissue is a source of multipotent stem cells. *Mol Biol Cell,* 2002, vol. 13, 4279-4295

- *Japanese Journal of Transfusion and Cell Therapy,* 2013, vol. 59 (3), 450-456